Europäisches Patentamt

European Patent Office

Office européen des brevets

(11) Publication number: **0 434 879 A1**

# EUROPEAN PATENT APPLICATION

(21) Application number: **89403491.7**

(22) Date of filing: **14.12.89**

(51) Int. Cl.⁵: **C12N 5/08**

The microorganism(s) has (have) been deposited with European collection of Animal cell cultures under number(s)89091401

(43) Date of publication of application:
**03.07.91 Bulletin 91/27**

(84) Designated Contracting States:
**FR**

(71) Applicant: **LABORATOIRES UNICET**
**92, rue Baudin**
**F-92307 Levallois-Perret(FR)**

(72) Inventor: **Banchereau, Jacques**
**25 Avenue Paul Santy**
**F-69130 Ecully(FR)**
Inventor: **Rousset, Françoise**
**126 rue Commandant Charcot**
**F-69005 Lyon(FR)**

(74) Representative: **Durand, Yves Armand Louis**
**et al**
**CABINET WEINSTEIN 20, Avenue de**
**Friedland**
**F-75008 Paris(FR)**

(54) Method of making factor dependent human B cell lines.

㊗ A method is provided for establishing factor dependent human B cell lines capable of secreting immunoglobulin of a desired specificity and capable of long term culturing. The method includes selecting a resting B cell having immunoglobulin of the desired specificity and culturing it in the presence of an agent capable of cross-linking its CD40 surface antigens. Long term culture requires the continued presence of the cross-linking agent. Preferably, the cross-linking agent is a monoclonal antibody specific for the CD40 antigen, presented by non-replicating mammalian cells expressing Fc$_\gamma$-RII.

# METHOD OF MAKING FACTOR DEPENDENT HUMAN B CELL LINES

## Field of the invention

The invention relates generally to the field of immunology, and more particularly, to methods of establishing longterm cultures of human lymphoid B cells capable of producing antibodies.

## BACKGROUND

Readily available hybridomas producing human monoclonal antibodies would give rise to valuable pharmaceutical and diagnostic compositions. Areas where human mnoclonal antibodies may prove directly useful include passive immunization against viral and bacterial diseases, elimination of drugs and toxins, diagnostic imaging of neoplasms, targeting of drugs to tumors, and modulation of autoimmune disorders. Indirect utility of human monoclonal antibody-producing hybridomas lies with their use as a source of messager RNA for making genetically engineered monoclonal antibodies in bacteria, or other non-human expression Systems, e.g. Skerra et al, Science, Vol. 240, pgs. 1038-1041 (1988); and Moore et al, U.S. patent 4,642,334. Such methods have the great advantage of providing antibodies or binding compositions free of potentially dangerous human contaminants. Unfortunately, to date the use of human monoclonal antibodies in in vivo trials has been very limited, e.g. Burnett et al, in Strelkauskas, ed. Human Hybridomas: Diagnostic and Therapeutic Applications (Marcel Dekker, New York, 1987). A major stumbling block to progress in the field has been the inability to obtain long term and/or immortalized human B cell lines, e.g. James et al, J. Immunol. Meth., Vol. 100, pgs. 5-40 (1987); and Van Brunt, Biotechnology, Vol. 7, pgs. 561-563 (1989).

The availability of methods for routinely producing such lines, and methods of enriching and expanding antigen-specific subpopulations of B cells, would be a major breakthrough for the application of human monoclonal antibodies.

## SUMMARY OF THE INVENTION

The invention is directed to a method of establishing factor-dependent human B cell lines and antigen-specific subpopulations. The method includes the steps of isolating resting B cells or antigen-specific subpopulation carrying immunoglobulin of a desired specificity, and culturing the B cell or antigen-specific subpopulation in the presence of an agent capable of cross-linking its CD40 antigens. Preferably, the cross-linking agent is an immobilized monoclonal antibody specific for CD40. More preferably, the monoclonal antibody is immobilized on a solid phase or non-aqueous phase liquid substrate, such as microspheres, liposomes, or cellular membranes. Most preferably, the anti-CD40 monoclonal antibody is immobilized by culturing the B cell or antigen-specific subpopulation with non-replicating mammalian cells expressing the surface molecule, CDw32, also known as Fc$\gamma$RII, whenever the CD40-specific monoclonal antibody is of the IgG isotype. In this case, immobilization is achieved by the binding of the Fc portion of the antibody molecules with the Fc$\gamma$ receptor. Longterm culturing of the B cell clone or subpopulation requires the continued presence of a cross-linking agent, and the culture growth rate is enhanced by the presence of the cytokines interleukin-2 (IL-2), interleukin-4 (IL-4), interleukin-6 (IL-6), and interferon-$\gamma$ (INF-$\gamma$), either alone or in combination.

The invention also includes a method for producing Epstein-Barr virus (EBV)-transformed B cell lines wherein transformation takes place in the presence of a CD40 cross linking agent.

An important feature of the invention is the use of resting B cells as a starting material. These cells, as opposed to activated B cells, retain their surface-bound immunoglobulin, thereby rendering them amenable to antigen-specific selection.

## Brief description of the drawings

Figure 1A and 1B illustrate data on the growth of B cells on feeder layers of irradiated L cells expressing CDw32 with and without anti-CD40 monoclonal antibody and with and without IL-4.

Figure 2 illustrates the growth of resting B cells in response to anti-CD40 antibody presented in different ways.

## DETAILED DESCRIPTION OF THE INVENTION

Resting B cells for use in the invention can be obtained from a variety or sources by a variety of means, e.g. DiSabato et al. eds., Meth. in Enzymol., Vol. 108 (1984), and James et al (cited above). Preferably, B cells are obtained from peripheral blood, spleen, or tonsils. Most preferably, B cells are obtained from tonsils using the following technique: Tonsils are dissociated with wire mesh in phosphate buffered saline, pH 7.2, to obtain single cell suspensions. Mononuclear cells are separated by the standard Ficoll-Hypaque gradient method. To obtain purified B cell populations, T cells are removed from the mononuclear cells by twice rosetting with 2-aminoethylisothiouronium bromide treated sheep erythrocytes. Adherent cells (monocytes) are removed from the T cell-depleted mononuclear cells by incubating T cell-depleted mononuclear cells (in batches of approximately $2.5 \times 10^8$ cells) in plastic flasks containing 25 ml RPMI 1640 with 10% fetal calf serum for 1 hour at 37°C. As determined by fluorescent activated cell sorting analysis the resulting preparation contains > 98% B cells, < 1% T cells, and < 1% monocytes. Resting B cells are obtained from this preparation by using a discontinuous gradient of Percoll (Pharmacia, Uppsala, Sweden) consisting of four solutions with densities of 1.075, 1.070, 1.060, and 1.055 g/ml. Resting B cells are recovered in the pellet, below the solution of Percoll of the highest density.

Further selection for antigen-specific subpopulation of resting B cells can be carried out by a variety of techniques including panning, rosetting, immunoadsorbent affinity chromatography, fluorescent-activated cell sorting (FACS), and the like. Casali et al, Science, Vol. 234, pgs. 476-479 (1986), describe the selection of antigen-specific 8 cells from peripheral blood by fluorescent-activated cell sorting. Briefly, the antigen of interest is biotinylated, incubated with the B cells, then with fluorescently labeled avidin. Cells having antibodies specific for the biotinylated antigen are sorted by the presence of the fluorescent label. Additional references describing FACS-based lymphocyte selection include Parks et al, Meth. Enzymol., Vol. 108, pgs. 197-241 (1984); and U.S. patent 4,325,706. Panning, immunoadsorbent affinity chromatography, and rosetting are described by Mage, Hubbard et al, and Haegert in Meth. Enzymol., Vol. 108, pgs. 118-124, 139-147, and 386-392, respectively (1984).

Monoclonal antibodies specific for CD40 are obtained by standard methods. Preferably, monoclonal antibodies G28-5 or Mab 89 are used as cross-linking agents. G28-5 is described by Ledbetter et al, J. Immunol., Vol. 138, pgs. 788-794 (1987) and in United Kingdom patent application N° 8713650, and the hybridoma cell line producing monoclonal antibodies G28-5 is deposited at the American Type Culture Collection (ATCC) (Rockville, MD) under accession number HB 9110. Mab 89 is described in Valle et al. Eur. J. Immunol., Vol. 19, pgs. 1463-1467 (1989), and the hybridoma cell line producing monoclonal antibodies Mab 89 is deposited with the European Collection of Animal Cell Cultures, PHLS Centre for Applied Microbiology and Research, Porton Down, Salisbury, Wilts. SP4 OJG, U.K. under accession number 89091401. Briefly, Mab 89 was obtained as follows. Eight week old BALB/c mice were injected i.p. four times at 3 week intervals with $5.0 \times 10^6$ anti-IgM antibody activated tonsillar B cells. Three days after the last injection, spleen cells were collected and fused with NS1 myeloma cells (ratio 5:1) with the use of polyethylene glycol 1000 (Merck). After overnight incubation at 37°C in a 50 ml flask in complete RPMI 1640 medium containing 10% heat inactivated fetal bovine serum, 2mM glutamine, 100 IU/ml penicillin and 100 μg/ml streptomycin, the cell suspension was distributed in 24-well plates in medium supplemented with hypoxanthine and azaserine. Hybridoma supernatants were screened for their ability to bind to Jijoye cells, tonsil mononuclear cells and anti-IgM antibody activated B cells.

Several different substrates can be used to immobilize the anti-CD40 monoclonal antibodies, such as microspheres, erythrocytes, irradiated hybridomas expressing surface anti-CD40, and the like. Preferably, mammalian cell lines capable of stable expression of the Fc$\gamma$R are produced by co-transfecting a host mammalian cell with a vector carrying a selectable marker and a vector carrying a host-compatible promoter and a cDNA insert capable of encoding Fc$\gamma$RII. A cDNA clone carrying such an insert, pcD-hFc$\gamma$-16.2, is available from the ATCC under accession number 67565, and is described in Stuart et al, J.Exp. Med., Vol. 166, pgs. 1668-1684 (1987). The vector is similar to the pcD shuttle vector described by Okayama and Berg, Mol. Cell. Biol., Vol. 2, pgs. 161-170 (1982), and Vol. 3, pgs. 280-289 (1983), except that the SV40 promoter has been modified to improve expression by the down stream insertion of a portion of the long terminal repeat from a HTLV(I) retrovirus, as described by Takebe et al, Mol. Cell. Biol., Vol. 8, pgs. 466-472 (1988). The vector is conveniently propagated in E. coli K12 strain MC1061, described in J. Mol. Biol., Vol. 138, pg. 179 (1980).

For pcD-hFc$\gamma$R-16.2, hosts include Chinese hamster ovary cells and mouse L cells, such as a thymidine kinase deficient mutant (tk⁻) L cell available from the American Type Culture Collection under accession number CCL 1.3. The selectable marker allows one to select host cells which have a high probability of containing the Fc$\gamma$R gene fully integrated into the host genome. Typically, the ratio of pcD-hFc$\gamma$R-16.2 to the marker containing vector in the transfection solution is about 10:1. Thus, if the marker gene is integrated into the host genome, it is very likely that pcD-hFc$\gamma$R-16.2 will also be integrated by virtue of its higher concentration. The selectable marker also provides a means of preventing the cultures of

3

desired transformants from being overgrown by revertant cells. tk⁻ mouse L cells were cotransfected with pcD-hFc$\gamma$R-16.2 and pSV2tk, a pSV2 plasmid carrying a thymidine kinase gene under control of the SV40 early promoter. The pSV2 is described in Mulligan et al., Science, Vol. 209, pgs. 1422-1427 (1980); Subramani et al. Mol. Cell. Biol., Vol. 1, pgs. 854-864 (1981); and is available from the American Type Culture Collection under accession number 37146. Both plasmids are amplified in E. coli, e.g. strain HB101 available from the ATCC under accession number 33694, and purified by cesium chloride equilibrium centrifugation. A suspension of about $1 \times 10^5$ of tk⁻ L cells in 10 ml of Dulbecco's Modified Eagle medium (DME) with 10% fetal bovine serum is placed in a Falcon 3003 dish and cultured at 37°C for 20 hours in a 5% carbon dioxide gas incubator, after which the medium is replaced by 10 ml of fresh DME with 10% fetal bovine serum. The culture is incubated for an additional 4 hours. After incubation 0.5 ml of soluble A (50 mM Hepes, 280 mM NaCl, 1.5 mM sodium phosphate buffer, pH 7.22) and 0.5 ml of solution B (2M CaCl₂, 10 mg pcD-Fc$\gamma$R-16.2, 1 mg pSV2tk) are added to the culture medium, and the culture is incubated at 37°C for 24 hours in a 5% CO₂ astmosphere, after which the cells are placed in a selective medium with HAT (e.g. Sigma Chemical Co., St. Louis, MO). After two weeks the surviving colonies are subcloned by limiting dilution, and clones are assayed for expression of Fc$\gamma$R.

Preferably, IL-2, IL-4, IL-6, or INF-$\gamma$ either alone or in combination, is added to the B cell culture at a concentration of about 1 nanomolar. Alternatively, the concentration of IL-4 may be expressed in terms of units/ml, where units are defined as in Yokota et al, Proc. Natl. Acad. Sci., Vol. 83, pgs. 5894-5898 (1986). There a unit of IL-4 is defined as the amount of IL-4 required to cause half-maximal stimulation of tritiated thymidine uptake by $5 \times 10^3/200 \mu l$ T cells which were preactivated for 3 days with phytohemagglutinin and then extensively washed. Preferably, B cell cultures include about 100 U/ml of IL-4.

## EXAMPLES

Example 1. Longterm Culture of Human B Cells Dependent on anti-CD40 Antibody and IL-4

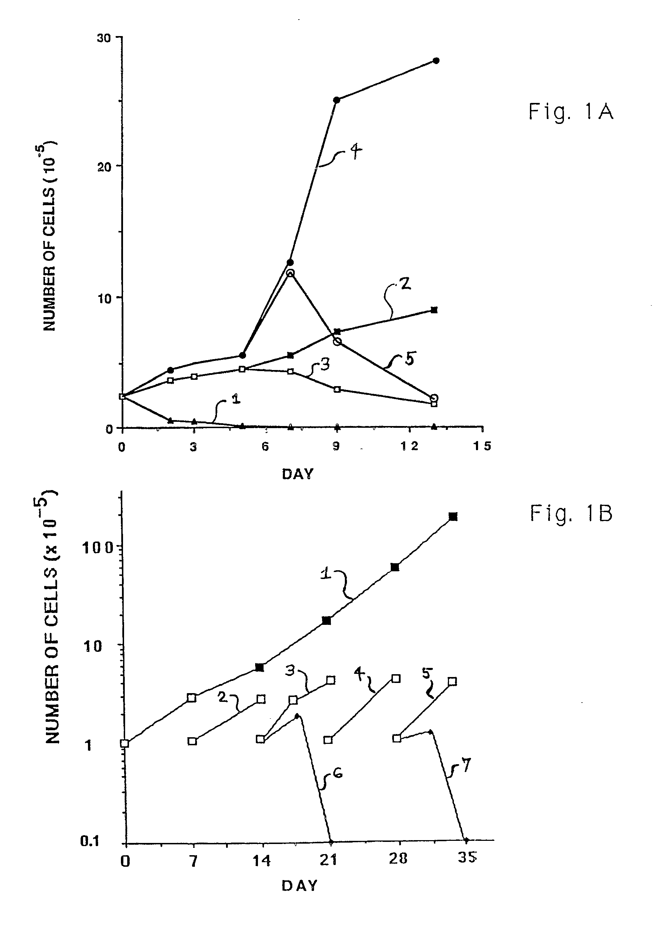

$2 \times 10^5$ purified spleen B cells in 500 $\mu l$ complete culture medium were seeded in wells of 48-weil microplates containing $2.5 \times 10^4$ irradiated CDw32 L cells with 1 $\mu g/ml$ anti-CD40 Mab 89 with or without 100 U/ml IL-4. Cultures inoubated with either Mab 89 or Mab 89 plus IL-4 were divided at day 5 into two wells plated at $2.5 \times 10^4$ irradiated CDw32 L cells with or without the original stimulant. Enumeration of viable B cells using Trypan Blue exclusion was carried out on a haemocytometer. Figure 1A shows the degree of B cell population growth for each culture condition. Cell numbers counted in one weil at days 7, 9 and 13 have been doubled to take into account the splitting of the culture at day 5. The cell numbers have been evaluated in ten identical wells and the coefficient of variation was found to be less than 10%. Curve 1 of Fig. 1A illustrates the growth of B cells cultured on CDw32 L cells without Mab 89 and without IL-4. Curve 2 illustrates the growth of B cells cultured initially with 1 $\mu g/ml$ Mab 89, then at day 5 the culture was split and half of the cells was transferred to another weil with $2.5 \times 10^4$ irradiated CDw32 L cells with 1 $\mu g/ml$ Mab 89. Curve 3 illustrates the growth of B cells cultured initially with 1 $\mu g/ml$ Mab 89, then at day 5 the culture was split and half of the cells was transferred to another weil with $2.5 \times 10^4$ irradiated CDw32 L cells without Mab 89. Curve 4 illustrates the growth of B cells cultured initially with 1 $\mu g/ml$ Mab 89 and 100 U/ml IL-4, then at day 5 the culture was divided and half of the cells was transferred to another well with $2.5 \times 10^4$ irradiated CDw32 L cells and 1 $\mu g/ml$ Mab 89 and 100 U/ml IL-4. Curve 5 illustrates the growth of B cells cultured initially with 1 $\mu g/ml$ Mab 89 and 100 U/ml IL-4, then at day 5 the culture was divided and half of the cells was transferred to another weil with $2.5 \times 10^4$ irradiated CDw32 L cells but without either Mab 89 or IL-4. The enhancing effect of CD40 cross linking in the presence of IL-4 is clearly seen.

In a separate experiment, purified tonsillar B cells were cultured at $10^5$ cells/ml on irradiated CDw32 L cells and 100 U/ml IL-4 in 500 $\mu l$ of complete medium in wells of 48-well microplates. Cells were enumerated at the indicated times shown on Fig. 1B and cultures were re-initiated at the end of each week by seeding $10^5$ B cells into new wells containing freshly irradiated CDw32 L cells, and fresh medium containing 1 $\mu g/ml$ Mab 89 and 100 U/ml IL-4. Curve 1 illustrates the theoretical B cell population size if depleted culture media did not have to be replaced. Curves 2 through 5 illustrate the actual B cell populations in the initial culture and in the re-initiated cultures. Curves 6 and 7 show the decline in population size when a sample of B cell culture is re-initiated without Mab 89.

Example 2 Further enhancement of B cell growth by IL-6 and IFN-$\gamma$

The growth enhancing effects of various cytokines were tested. $5 \times 10^3$ purified tonsillar B cells were cultured on $5 \times 10^3$ irradiated CDw32 L cells with 1 $\mu g/ml$ Mab 89 in conical microwells. Tritiated thymidine uptake (after a 16 h pulse with 1 $\mu$Ci) was assayed at the time points indicated in the table below. Each value in the table is a means of triplicate determinations. Cytokine concentrations were 25 U/ml IL-4; 2.5 IU/ml IL-1; 20 IU/ml IL-2; 50 U/ml IL-6; and 1000 IU/ml IFN-$\gamma$. A strong synergistic growth-inducing effect is seen between IL-4 and IFN-$\gamma$ at day 8.

Table I

| Cytokine(s) Added | Tritiated Thymidine Uptake (cpm x $10^{-3}$) | | | |
|---|---|---|---|---|
| | Day 2 | Day 4 | Day 6 | Day 8 |
| -- | 1.5 | 5.3 | 7.0 | 5.1 |
| IL-4 | 3.1 | 7.9 | 13.3 | 21.8 |
| IL-2 | 2.3 | 4.4 | 12.7 | n.d. |
| IL-6 | 1.1 | 9.3 | 25.0 | 13.0 |
| IFN-$\gamma$ | 4.6 | 6.7 | 16.6 | 14.0 |
| IL-4 + IL-2 | 1.8 | 13.2 | 18.6 | 18.4 |
| IL-4 + IL-6 | 1.9 | 17.8 | 22.8 | 32.0 |
| IL-4 + IFN-$\gamma$ | 4.9 | 21.5 | 34.1 | 60.9 |

Example 3. Antibody production by B cells stimulated with Mab 89 presented on CDw32-transfected L cells

$2.5 \times 10^5$ purified B cells were cultured with or without $2.5 \times 10^4$ irradiated CDw32 L cells, with or without 0.5 $\mu$g/ml Mab 89, and with or without 100 U/ml IL-4. Supernatants were harvested at day 8 and immunoglobulin concentrations were determined by ELISA. The results for the various isotypes are shown in Table II.

Table II

| Culture Conditions | Concentration (ng/ml) | | | |
|---|---|---|---|---|
| | IgG | IgA | IgM | IgE |
| Control | 80 | 20 | 125 | <0.2 |
| Mab 89 | 225 | 70 | 380 | -- |
| IL-4 | 100 | <10 | 80 | <0.2 |
| Mab + IL-4 | 105 | 30 | 170 | <0.2 |
| L cells | 210 | 10 | 290 | <0.2 |
| L cells + IL-4 | 650 | 200 | 490 | <0.2 |
| L cells + Mab 89 | 1800 | 40 | 320 | <0.2 |
| L cells + Mab 89 + IL-4 | 5825 | 650 | 31200 | 458 |

Example 4. Stimulation of B cell growth by irradiated hybridoma 89 cells

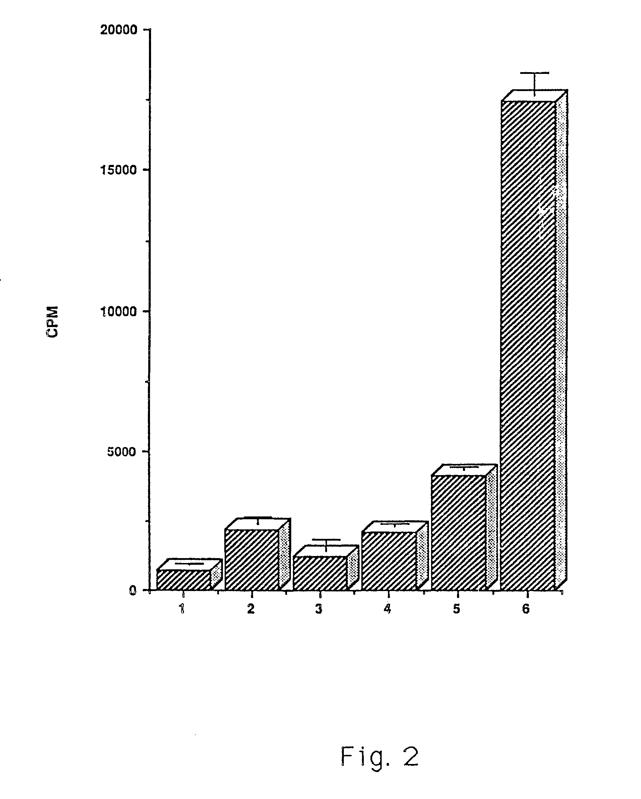

The growth-inducing effect of a hybridoma expressing surface anti-CD40 antibody was tested. Six experimental conditions were examined: a control consisting of microtiter plate wells seeded with $10^5$ purified B cells; a control consisting of microtiter plate wells seeded with $10^5$ purified B cells and 1 $\mu$g/ml Mab 89; a control consisting of microtiter plate wells seeded with $10^4$ irradiated hybridoma 89.1.4 cells (this is a derivative of hybridoma 89 which expresses antibody on its surface); a control consisting of microtiter plate wells seeded with $10^5$ purified B cells and $10^4$ cells of an irradiated and unrelated hybridoma; a control consisting of microtiter plate wells seeded with $10^5$ purified B cells and $10^4$ irradiated hybridoma 89.1.4 cells; and a control consisting of microtiter plate wells seeded with $10^5$ purified B cells and $10^4$ irradiated CDw32 transfected L cells and 1 $\mu$g/ml of Mab 89. Cell growth under the respective conditions

5

was assayed by tritiated thymidine incorporation. The results are shown in Figure 2. Columns 1 through 6 on the Figure correspond to the conditions listed above, respectively. The immobilized anti-CD40 on irradiated hybridoma 89.1.4 is nearly as effective as that on CDw32 transfected L cells in inducing growth in resting B cells.

Example 5. Enhancement of EBV infection of B cells by Mab 89

An important way to establish antibody-producing human B cell lines is by infecting the B cells with Epstein Barr virus (EBV), e.g. James, Scand. J. Immunol., Vol. 29, pg. 257 (1989). However, the infection procedure is very inefficient, e.g. Stein et al. Cell. Immunol., Vol. 79, pg. 309 (1983). It was discovered that the presence of a CD40 cross-linking agent increases the efficiency of B cell infection. Infection efficiency was measured as a function of initial B cell numbers and the presence or absence of Mab 89, IL-4, or IL-6. Cultures were performed in 48-well (flat bottoms) plates for $10^5$-cell cultures (each also containing $2.5 \times 10^4$ irradiated CDw32 L cells) and in 96-well (round bottoms) plates for 100-cell and single cell colonies (each also containing $5.10^3$ irradiated CDw32 L cells). Factor concentrations were as follows: 1 $\mu$g/ml of Mab 89, 100 U/ml of IL-4, and 50 U/ml of IL-6. Table III lists the fraction of wells from which continuous EBV-transformed B cell lines were obtained for the various starting conditions and culture conditions.

Table III

| Culture Conditions (Item added): | | | | Initial Cell Number (wells with transformants/total number of wells) | | |
|---|---|---|---|---|---|---|
| L cells | Mab 89 | IL-4 | IL-6 | $10^5$ | 100 | 1 |
| - | - | - | - | 0/48 | 0/192 | 0/288 |
| + | - | - | - | 48/48 | 0/192 | 1/288 |
| + | + | - | - | 48/48 | 29/192 | 8/288 |
| + | + | + | - | 48/48 | 8/192 | 0/288 |
| + | + | - | + | 48/48 | 16/192 | 4/288 |
| + | + | + | + | 48/48 | 11/192 | 2/288 |

Besides enhanced EBV infection, the Mab 89/L cell System also affects antibody production by the EBV infected B cells. $10^5$ resting B cells were cultured on $2.5 \times 10^4$ irradiated L cells expressing CDw32 and 1 $\mu$g/ml Mab 89 and EBV. Cells were enumerated at day 8 and immunoglobulin levels were measured by ELISA. Table IV lists the results and shows that the cells produce very high levels of IgE under these conditions.

Table IV

| Culture conditions | Cell Number x $10^5$ | Concentration (ng/ml) | | | |
|---|---|---|---|---|---|
| | | IgG | IgA | IgM | IgE |
| L cells + EBV | 0.45 | 667 | 105 | 959 | <0.2 |
| L cells + Mab 89 + EBV | 3.7 | 1245 | 639 | 1717 | <0.2 |
| L cells + Mab 89 + EBV + IL-4 | 6 | 683 | 87 | 572 | 478 |

Example 6. Establishment of Human B Cell Clone Producing Rh Factor-Specific Monoclonal Antibodies

An important clinical use of anti-Rh antibodies is their injection into Rh-negative women shortly after the delivery of an Rh-positive infant to prevent the development of indigenious anti-Rh antibodies by the women which could be harmful to infants of subsequent pregnancies, e.g. Crookston, pgs. 601-608, in Rose et al, eds., Manual of Clinical Laboratory Immunology, 3rd Ed. (American Society for Microbiology, Washington, D.C., 1986). Besides the cost and difficulty of obtaining suitable donors for the anti-Rh immunoglobulin, a frequent danger associated with such injections is the transmission of blood-borne diseases such hepatitis, AIDS, and the like. In view of this problem, an in vitro source of anti-Rh antibodies would be highly desirable.

Resting B lymphocytes are isolated from an Rh-negative donor having serum containing anti-Rh antibody. A subpopulation of resting B cells carrying anti-Rh surface immunoglobulin may be isolated by repeated panning of the isolated B lymphocytes by the technique described by Mage (cited above), modified in the following manner: Instead of using a tissue culture dish coated with antibody specific for the anti-Rh antibody, the tissue culture dish is coated with anti-biotin antibody, and prior to addition to the tissue culture dish the isolated B lymphocytes are incubated with biotinylated Rh antigen. Cells binding the biotinylated Rh antigen are then isolated by panning as described by Mage (cited above). Alternatively, the resting B lymphocytes are isolated by fluorescent-activated cell sorting (FACS) using fluorescently labeled Rh antigen, or by rosetting following standard procedures, e.g. Elliott et al, Meth. Enzymol., Vol. 108, pgs 49-64 (1984).

The isolated anti-Rh resting B cells are then distributed among the wells (about $10^5$ B cells per well) of a microtiter plate, each well having previously been seeded with about $10^4$ CDw32-transfected, irradiated L cells, and each well containing 0.4 ml of medium (RPMI 1640 with 10% fetal calf serum, and 2 mM glutamine) further containing 0.5 mg/ml of Mab 89 and 100 U/ml of IL-4. Cultures are expanded into larger containers and harvested for anti-Rh antibody after several weeks.

## Claims

1. A method for making human B cell lines, the method comprising the steps of:
isolating a human resting B cell having CD40 antigens and having immunoglobulin of a desired specificity; and
culturing the human resting B cell in the presence of an agent capable of cross-linking CD40 antigens.

2. The method of claim 1 wherein said cross-linking agent is an immobilized monoclonal antibody specific for said CD40 antigen.

3. The method of claim 2 wherein the step of culturing further includes culturing in the presence of interleukin-4 and/or interferon-$\gamma$.

4. The method of claim 3 wherein said immobilized monoclonal antibody is attached to FC$\gamma$RII receptors expressed by non-replicating mammalian cells.

5. The method of claim 4 wherein said monoclonal antibody is selected from the group consisting of Mab 89 and G28-5 and wherein said non-replicating mammalian cells are mouse L cells stably transformed by pcD-hFc$\gamma$R-16.2.

6. A method of producing Epstein-Barr virus-transformed human B cells, the method comprising the step of culturing human B cells in the presence of Epstein-Barr Virus and an agent capable of cross-linking CD40 antigens.

7. The method of claim 6 wherein said agent is an immobilized monoclonal antibody specific for said CD40 antigen.

8. The method of claim 7 wherein said immobilized monoclonal antibody is attached to Fc$\gamma$RII receptors expressed by non-replicating mammalian cells.

9. The method of claim 8 wherein said monoclonal antibody is selected from the group consisting of Mab 89 and G28-5 and wherein said non-replicating mammalian cells are mouse L cells stably transformed by pcD-hFc$\gamma$R-16.2.

Fig. 1A

Fig. 1B

Fig. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | BIOLOGICAL ABSTRACTS, vol. 87, no. 12, 1989, abstract no. 127968, Philadelphia, PA, US; J.L. LASKY et al.: "Characterization and growth factor requirements of SJL lymphomas: II. Interleukin 4 dependence of the in vitro cell line, cRCS-X, and influence of other cytokines", & EUR. J. IMMUNOL. 19(2): 365-372. 1989 * Abstract * | 1 | C 12 N 5/08 |
| Y | BIOLOGICAL ABSTRACTS, vol. 79, no. 11, 1985, abstract no. 95695, Philadelphia, PA, US; R. FRADE et al.: "Enhancement of human B cell proliferation by an antibody to the complement C3d receptor, the glycoprotein gp1 40 molecule", & EUR. J. IMMUNOL. 15(1): 73-76. 1985 * Abstract * | 1 | |
| Y | THE JOURNAL OF IMMUNOLOGY, vol. 142, no. 5, 1st March 1989, pages 1569-1575, The American Association of Immunologists, US; J.B. SPLAWSKI et al.: "Immunomodulatory role of IL-4 on the secretion of Ig by human B cells" * The whole article * | 1 | **TECHNICAL FIELDS SEARCHED (Int. Cl.5)**<br><br>C 12 N |

The present search report has been drawn up for all claims

| Place of search | Date of completion of the search | Examiner |
|---|---|---|
| THE HAGUE | 06-07-1990 | REMPP G.L.E. |